# EUROPEAN PATENT APPLICATION

(11) **EP 4 471 143 A1**
(43) Date of publication of application: **04.12.2024**
(21) Application number: 23382510.8
(22) Date of filing: 30.05.2023
(51) Int. Cl.: C12N 15/113, A61K 31/00, A61K 31/7088

(54) **MIR-148A FOR USE IN THE STIMULATION OF T-CELL ANTI-TUMOR RESPONSE**

(71) Applicant: Universidad Autónoma de Madrid, 28049 Madrid (ES)
(72) Inventor: GONZÁLEZ MARTÍN, Alicia, 28049 Madrid (ES); GONZÁLEZ MOLINA, María del Pilar, 28049 Madrid (ES)
(74) Representative: Hoffmann Eitle

(57) **Abstract**

The present invention refers to the medical field. Particularly, the present invention refers to the miRNA miR-148a, a miR-148a mimic, a genetic construct encoding miR-148a and/or a viral vector encoding miR-148a for use in the stimulation of T-cell anti-tumor response. It also refers to a composition comprising thereof for use in the stimulation of T-cell anti-tumor response.

## Description

### FIELD OF THE INVENTION

The present invention refers to the medical field. Particularly, it refers to the miRNA miR-148a, a miR-148a mimic, a genetic construct encoding miR-148a and/or a viral vector encoding miR-148a for use in the stimulation of T-cell anti-tumor response. It also refers to a composition comprising thereof for use in the stimulation of T-cell anti-tumor response.

### STATE OF THE ART

Recent advances in tumor immunology research have enabled the development of a series of cancer immunotherapy strategies, including checkpoint inhibitors and adoptive-cell transfer. Cancer immunotherapy holds the promise of permanently curing a large variety of tumors of different origins. However, these therapies are effective in only a subset of cancer patients and not in others. This implies the need to identify new targets that allow the development of improved cancer immunotherapy strategies for those patients that do not respond or respond poorly to the current treatments.

Most research in the tumor immunology field has focused on protein coding genes. However, protein coding genes account for less than 2% of the human genome. Among the different noncoding RNAs that have been annotated, microRNAs (miRNAs) are the most attractive targets to enhance antitumor immunity due to their intrinsic capacity to control cell functions fast and efficiently through the simultaneous downregulation of a network of cell signalling pathways that regulate cell proliferation, apoptosis and effector functions, among other processes. MiRNAs are 19-23 nucleotides long RNAs that regulate the expression of their target genes by pairing through imperfect sequence complementarity with their target messenger RNAs (mRNAs) and promoting mRNA degradation and/or translational repression. Approximately 2500 different human microRNAs have been identified, some of which have been shown to play important roles in the regulation of the immune response. However, the roles that miRNAs play in the specific context of antitumor immunity remain largely unexplored with only a few miRNAs previously reported to regulate this process. Biotechnological companies are developing microRNAs-based therapeutics for multiple human diseases, with some compounds already advancing in clinical trials. This provides the possibility of using miRNAs to improve the efficiency of the current cancer immunotherapies to benefit a larger number of patients.

So, there is an unmet medical need to identify new targets that allow the development of improved cancer immunotherapy strategies for those patients that do not respond to current treatments. The present invention is focused on solving this problem and a miRNA for the stimulation of T-cell anti-tumor response is herein provided.

### DESCRIPTION OF THE INVENTION

### Brief description of the invention

The present invention refers to miR-148a, a miR-148a mimic, a genetic construct encoding miR-148a and/or a viral vector encoding miR-148a for use in the stimulation of T-cell antitumor response. It also refers to a composition comprising thereof for use in the stimulation of T-cell anti-tumor response.

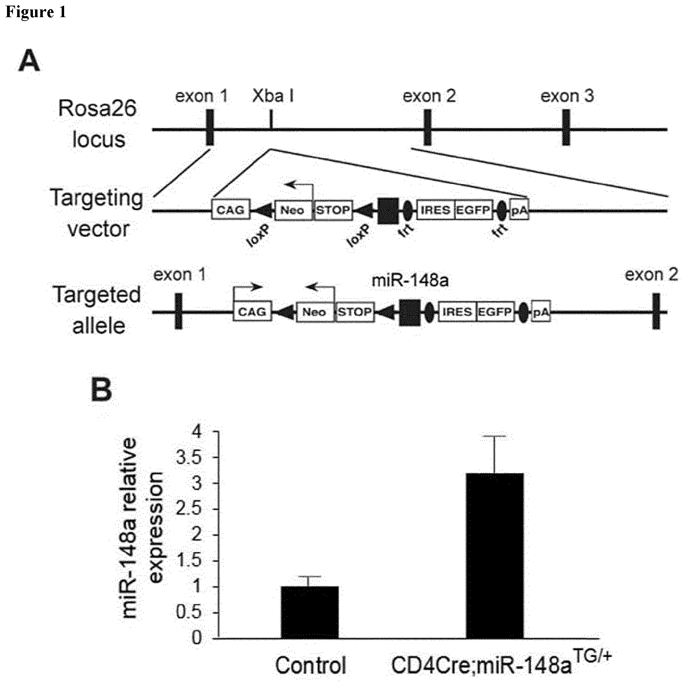

The inventors generated a transgenic mouse model for conditional miR-148a expression (**Figure 1A**). Expression of this transgenic allele requires removal of a STOP transcriptional cassette by Cre recombinase. To investigate the effects of miR-148a expression in T cells, mice carrying the miR-148a transgene were bred with mice expressing Cre recombinase under the control of the CD4 promoter. This resulted in the generation mice with the following genotypes:

| **Genotype name** | **Description** |
|---|---|
| Control | C57BL/6J wild type mice and mice expressing a CD4-Cre allele. |
| CD4Cre; miR-148a^{TG/+} or CD4Cre; miR-148a^{fl/+} | Mice expressing a CD4-Cre allele and one miR-148a transgenic allele. |
| CD4Cre; miR-148a^{TG/TG} or CD4Cre; miR-148a^{fl/fl} | Mice expressing a CD4-Cre allele and two copies of the miR-148a transgenic allele. |

The CD4-Cre transgene induces expression of Cre recombinase in early T cell development, specifically in CD25-CD44- double negative-stage-1 thymocytes. Thus, Cre-mediated excision of the 'Neo-STOP' cassette is expected to drive miR-148a expression in the T cell compartment. As expected, CD4Cre; miR-148a^{TG/+} mice displayed increased levels of miR-148a in splenic T cells compared to their control counterparts (**Figure 1B**).

The inventors then aimed to determine the effect of miR-148a on T-cell anti-tumor response, using lung cancer and melanoma as proof of concept.

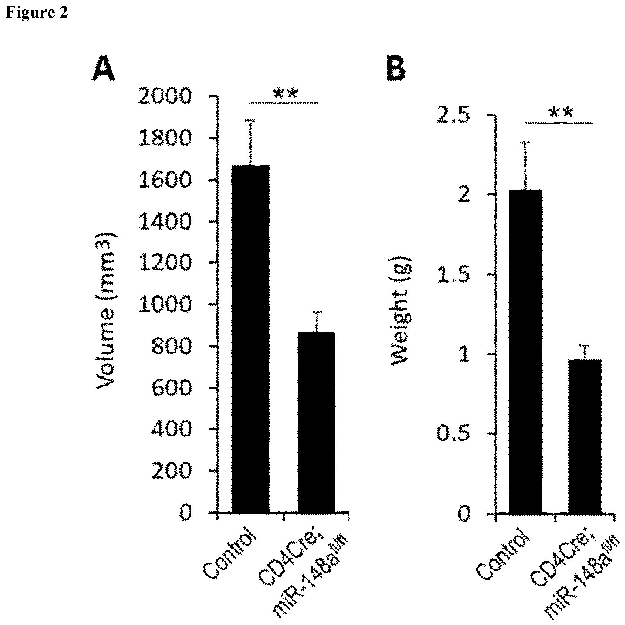

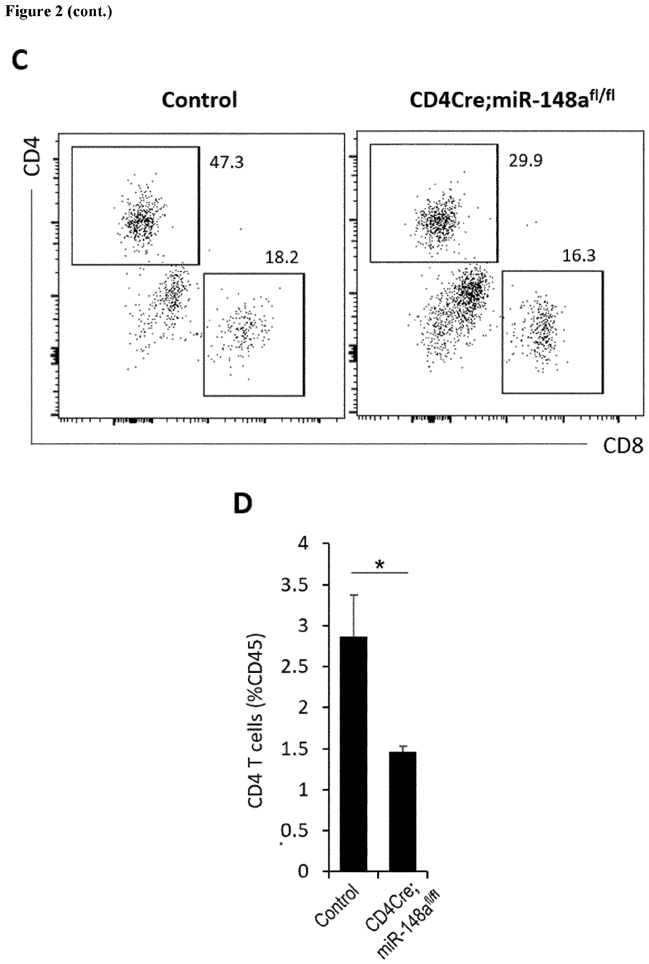

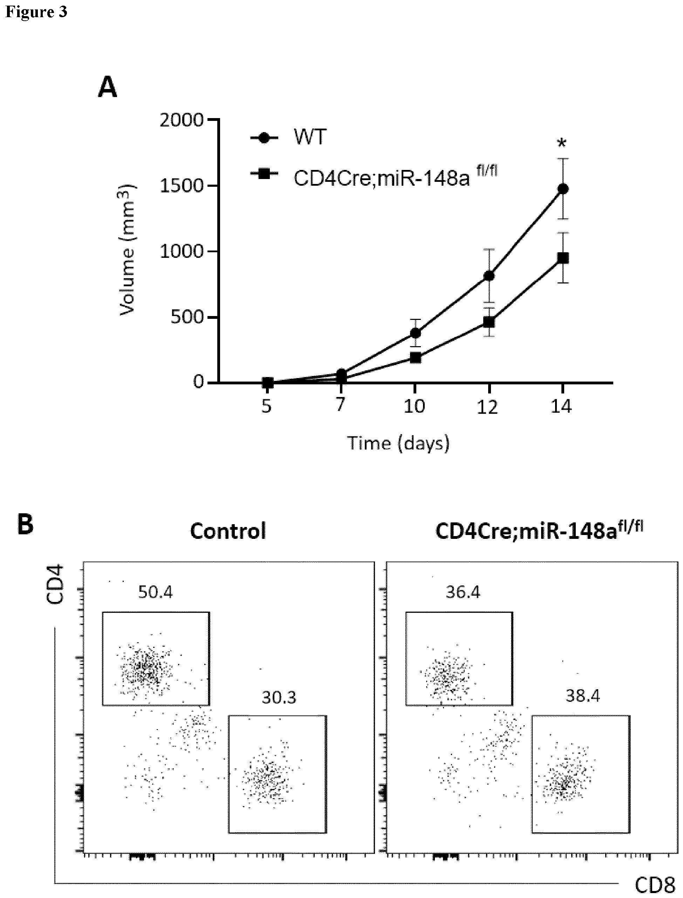

Increased expression of miR-148a in T cells was found to:
- Impair tumor growth (Figure 2A-B) and intratumoral CD4+ T infiltration in an experimental mouse model of lung cancer (LLC, Lewis lung carcinoma Figure 2C-D).
- Impair tumor growth in a preclinical mouse model of melanoma (**Figure 3A**) and intratumoral CD4+ T infiltration (Figure 3B-C) in a preclinical model of melanoma (B16-F10, Figure 3B-C).

Overall, these results indicate that miR-148a promotes T-cell anti-tumor response in mice.

The anti-tumoral effects associated to miR-148a were observed upon expression of the pre-processed miR-148 (SEQ ID NO: 1). However, miR-148a effector functions may be also ascribed to the mature miR-148a forms (SEQ ID NO: 2 and 3), which are generated upon a processing of the pre-miRNA (SEQ ID NO: 1) within the cell. The pre-processed miR-148a form (SEQ ID NO: 1) that comprises the mature forms (SEQ ID NO: 2 and 3), and mature miR-148a forms themselves, are summarised in **Table 1.**

**Table 1: Pre-processed and mature forms of miR-148a.**

| **SEQ ID NO** | **miRBase accession ID** | **Sequence name** | **Sequence** |
|---|---|---|---|
| SEQ ID NO: 1 | MI000025 3 | pre-miR-148a (pre-processed stem-loop sequence hsa-mir-148a) | GAGGCAAAGUUCUGAGA CACUCCGACUCUGAGUAU GAUAGAAGUCAGUGCACU ACAGAACUUUGUCUC |
| SEQ ID NO: 2 | MIMAT00 00243 | Mature sequence hsamiR-148a-3p | UCAGUGCACUACAGAACU UUGU |
| SEQ ID NO: 3 | MIMAT00 04549 | Mature sequence hsamiR-148a-5p | AAAGUUCUGAGACACUC CGACU |

So, the **first** embodiment of the present invention refers to miR-148a, a miR-148a mimic, a genetic construct encoding miR-148a and/or a viral vector encoding miR-148a for use in the stimulation of T-cell anti-tumor response.

In a preferred embodiment, the viral vector comprising the miR-148a gene is an adeno-associated viral vector, a retroviral vector or a lentiviral vector.

In a preferred embodiment, the miR-148a, miR-148a mimic, genetic construct encoding miR-148a and/or viral vector encoding miR-148a is administered alone or prior, after or concomitantly to the administration of a cancer treatment.

In a preferred embodiment, the miR-148a, miR-148a mimic, genetic construct encoding miR-148a and/or viral vector encoding miR-148a is administered alone or prior, after or concomitantly to the administration of a cancer treatment selected from the list comprising: checkpoint inhibitors, T-cell adoptive cell therapies, chemotherapy, radiotherapy, antibody-based cancer therapies, targeted-molecule inhibitors or small-molecule inhibitors.

In a preferred embodiment, the miR-148a, miR-148a mimic, genetic construct encoding miR-148a and/or viral vector encoding miR-148a is used in the treatment of lung cancer or melanoma.

In a preferred embodiment, the miR-148a, miR-148a mimic, genetic construct encoding miR-148a and/or viral vector encoding miR-148a gene is administered to the subject via intravenous administration, subcutaneous administration, intratumoral administration, or chemoembolization.

In a preferred embodiment, the genetic construct encoding miR-148a and/or viral vector encoding miR-148a is used to genetically modify T cells obtained from a patient, alone or in combination with other genetic modifications, to improve their antitumoral effect upon reinfusion into the patient.

Alternatively, hematopoietic stem and precursor cells (HSPCs), or another source of stem cells (for instance those derived from umbilical cord) can also be modified to overexpress miR-148a for their subsequent *in vitro* differentiation into T cells which would be infused into the patient. They could also be infused as miR-148a-overexpressing stem cells into immunocompromised patients to reconstitute their immune system.

The **second** embodiment of the present invention refers to a composition comprising miR-148a, a miR-148a mimic, a genetic construct encoding miR-148a gene and/or a viral vector encoding miR-148a gene for use in the stimulation of T-cell anti-tumor response.

In a preferred embodiment, the viral vector is an adeno-associated viral vector, a retroviral vector or a lentiviral vector.

In a preferred embodiment, the composition is administered alone or prior, after or concomitantly to the administration of a cancer treatment.

In a preferred embodiment, the composition is administered alone or prior, after or concomitantly to the administration of a cancer treatment selected from the list comprising: checkpoint inhibitors, T-cell adoptive cell therapies, chemotherapy, radiotherapy, antibody-based cancer therapies, targeted-molecule inhibitors or small-molecule inhibitors.

In a preferred embodiment, the composition is used in the treatment of lung cancer or melanoma.

In a preferred embodiment, the composition is administered to the subject via intravenous administration, subcutaneous administration, intratumoral administration, or chemoembolization.

In a preferred embodiment, the composition is a pharmaceutical composition comprising pharmaceutically acceptable excipients or carriers.

The miR-148a, miR-148a mimic, genetic construct encoding miR-148a, viral vector encoding miR-148a and/or any composition comprising thereof can be administered alone, or in any delivery vehicle contemplated in the common general knowledge, including liposomes, nanoparticles, transfection agents and nanoparticles.

The present invention also refers to a T cell or T cell-derived cell comprising or encoding miR-148a, a miR-148a mimic and/or a genetic construct encoding miR-148a. In a preferred embodiment, the T cells or T cell-derived cells are CAR-T cells presenting (the cell captures the mimic and once it is inside the cell, the mimic modulates gene expression), encoding or expressing the miR-148a or the mimic.

The invention also refers to a method for stimulating of T-cell anti-tumor response which comprises the administration of a therapeutically effective dose or amount of miR-148a, a miR-148a mimics, a genetic construct encoding miR-148a, a viral vector encoding miR-148a and/or any composition comprising thereof. In a preferred embodiment, the treatment is administered alone, prior, after or concomitantly to the administration of a cancer treatment, preferably selected from the list comprising: checkpoint inhibitors, T-cell adoptive cell therapies, chemotherapy, radiotherapy, antibody-based cancer therapies, targeted-molecule inhibitors or small-molecule inhibitors.

The invention also refers to a method for stimulating of T-cell anti-tumor response which comprises the administration of T cells or T cell-derived cells encoding and/or presenting miR-148a or a miR-148a mimic. In a preferred embodiment, the treatment is administered alone, prior, after or concomitantly to the administration of a cancer treatment, preferably selected from the list comprising: checkpoint inhibitors, T-cell adoptive cell therapies, chemotherapy, radiotherapy, antibody-based cancer therapies, targeted-molecule inhibitors or small-molecule inhibitors.

On the other hand, the inventors of the present invention propose to use synthetic locked nucleic acid (LNA) miR-148a mimics or genetic miR-148a expression alone or in combination with checkpoint inhibitors, tumor-specific T-cell adoptive transfer therapies, chemotherapy or targeted therapies to increase the efficiency of cancer therapies. LNA-modified miRNAs contain chemical modifications that results in increased affinity for their targets, stability, and resistance to degradation by nucleases. Their small size allows them to inhibit RNA targets inside tissues without the need for complex delivery vehicles, although delivery methods could be used to improve the efficacy of the therapy.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of". Thus, the phrase "consisting of' indicates that the listed elements are required or mandatory, and that no other elements may be present.
- The term "mimic" refers to a small RNA molecule designed to mimic a miRNA molecule either in its pre-processed or mature form having at least 80% sequence identity, resulting in the downregulation or translational repression of target mRNA translation due to mRNA sequestration or degradation. Mimics may optionally contain chemical modifications aimed at improving their binding affinity to complementary sequences, stability, and resistance to degradation by nucleases. This is the case of locked nucleic acid (LNA) mimics, which contain an O-methylene linkage between the 2 and 4 positions of the ribose, which leads to exceptionally high-affinity binding to complementary sequences.
- "Pharmaceutically acceptable excipient or carrier" refers to an excipient that may optionally be included with the pharmaceutical composition of the invention and that causes no significant adverse toxicological effects to the patient.

### Description of the figures

**Figure 1****. Generation of miR-148a conditional transgenic mice. A.** Strategy for the generation of conditional miR-148a transgenic mice by Rosa26 locus targeting. The miR-148a precursor preceded by the synthetic CAG promoter and a loxP-flanked 'Neo-STOP' cassette and followed by an IRES-GFP protein cassette (flanked with a Flp recombinase-recognition target) and the polyadenylation signal was knocked-in at the ubiquitously expressed Rosa 26 locus. **B.** Expression levels of miR-148a in splenic T cells of CD4Cre control and CD4Cre;miR-148a^{TG/+} mice as determined by miRNA Taqman PCR assays.
**Figure 2****. Increased expression of miR-148a in T cells impairs tumor growth and CD4 T cell infiltration into the tumor parenchyma in the Lewis lung carcinoma (LLC) mouse model.** LLC tumors were induced in control and CD4Cre;miR-148a^{TG/TG} (also referred to as CD4Cre;miR-148a^{fl/fl}) mice by subcutaneous inoculation of 0.5×10⁶ cells (LLC cells) into the right flank. After 22 days, mice were sacrificed, tumor size measured, and the tumor infiltrates characterized by flow cytometry. **A-B.** Tumor volume (**A**) and weight (**B**) of control and CD4Cre;miR-148a^{TG/TG}(or CD4Cre;miR-148a^{fl/fl}) at terminal analysis. **C.** Representative flow cytometry plot showing the percentage of tumor-infiltrating CD4⁺ and CD8+ T cells (gated in CD3⁺ T cells) as indicated at terminal analysis. **D.** Quantification of tumor-infiltrating CD4⁺ T cells in all mice analyzed in **C,** gated in the CD45⁺ immune tumor infiltrate, at terminal analysis. ^{∗}P ≤ 0.05, ^{∗∗}P ≤ 0.01.

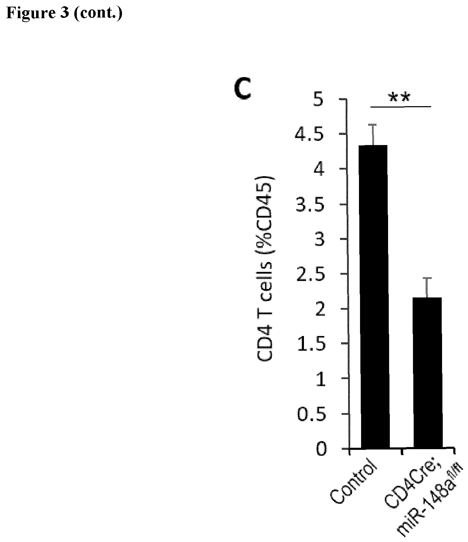

**Figure 3****. Elevated levels of miR-148a in T cells impairs tumor growth in the preclinical murine melanoma model B16-F10.** Melanoma tumors were induced in control and CD4Cre;miR-148a^{TG/TG} (or CD4Cre;miR-148a^{fl/fl}) mice by subcutaneous inoculation of 0.5×10⁶ B16-F10 cells into the right flank. Tumor growth was monitored over time. A. Graph shows tumor volume over the course of 14 days in the experimental groups with elevated miR-148a in T cells and control. B. Representative flow cytometry plot showing the percentage of tumor-infiltrating CD4⁺ and CD8⁺ T cells (gated in CD3⁺ T cells) as indicated at terminal analysis. C. Quantification of tumor-infiltrating CD4⁺ T cells in all mice analyzed, gated in the CD45⁺ immune tumor infiltrate, at terminal analysis. ^{∗}P ≤ 0.05, ^{∗∗}P ≤ 0.01.

### Detailed description of the invention

The present invention is illustrated by means of the Examples set below without the intention of limiting its scope of protection.

### Example 1. Material and Methods

### Mice

C57BL/6 Rosa26 knock-in conditional transgenic mice for miR-148a were generated using CRISPR/Cas9 gene targeting. For this, the sequence comprising human pre-miR-148a with flanking sequences of approximately 250 nucleotides in each side was be cloned into the targeting vector pR26 CAG/GFP Asc and used for mouse generation. Of note, the mature human and murine miR-148a share a 100% sequence homology and, thus, can be used indistinctively. The first litters were genotyped and characterized, and founder mice transferred to our institutional animal facility at IIBM to generate the mouse colony. These mice were bred with CD4Cre mice to obtain CD4Cre;miR-miR-148a^{TG/TG} mice with conditional increased expression of the miRNA in T cells.

### Tumor induction

Tumors were induced in age and gender-matched control and CD4Cre;miR-148a^{fl/fl} mice by subcutaneous inoculation of 5×10⁵ LLC or B16-F10 cells in the right flank. Both male and female mice were used in the experiments. In addition, both C57BL/6J wild-type and CD4Cre mice were included as controls. Tumor volume was measured twice or three times per week. When the tumors reached a volume of 1-2 cm³, mice were euthanized to analyze their tumor volume and weight, and their immune infiltration in the tumor parenchyma.

### Characterization of T cell infiltration at the tumor site.

To identify the specific cell/s subset/s responsible for the miR-148a-mediated control of antitumor immunity, an analysis tumor immune control and CD4Cre;miR-148a^{fl/fl} tumorbearing mice was performed. Innate and adaptive immune cell subsets were characterized, with a focus on T cell subsets including CD4 and CD8 T cells by flow cytometry. For this, tumors were mechanically disgregated into single-cell suspensions, erythrocytes were lysed by incubation with ACK lysis buffer 5min RT, and cells were stained with panels of antibodycoupled to fluorophores to detect specific cell markers including CD3, CD4, CD8, CD19, CD1 1b, Mac3 and Gr1. Stained cells were analyzed by flow cytometry in a BD FACSCanto II and a Beckman Coulter CytoFLEX S. The resulting data was analyzed using the Flowjo v10 software.

### Example 2. Results

### Example 2. 1. Generation of a transgenic mouse model with increased expression of miR-148a specifically in T cells

The inventors of the present invention have developed for the first time a C57BL/6J transgenic mouse model with increased expression of miR-148a specifically in T cells (CD4Cre; miR-148a TG). To generate miR-148a-transgenic (TG) mice, a genomic DNA fragment encoding miR-148a was cloned into a Rosa26 targeting vector (CTV). A genomic DNA fragment encoding the human miR-148a, preceded by the synthetic CAG promoter and a loxP-flanked 'Neo-STOP' cassette, was inserted into the ubiquitously expressed Rosa26 locus. Of note, the mature human and murine miR-148a sequence share a 100% sequence homology. An internal ribosomal entry site-enhanced green fluorescent protein (IRES-GFP) cassette flanked with a Flp recombinase-recognition target was placed between the miR-148a genomic DNA fragment and the polyadenylation signal (**Figure 1A**). Mice carrying this transgene were bred with mice transgenic for CD4-Cre to obtain mice expressing CD4-Cre and one copy of the miR-148a transgene (TG/+) or two copies of the miR-148a transgene (TG/TG, also referred to as fl/fl). The CD4-Cre transgene induces expression of Cre recombinase in early T cell development, specifically in CD25-CD44- double negative-stage-1 thymocytes. Thus, Cre-mediated excision of the 'Neo-STOP' cassette is expected to drive miR-148a expression in the T cell compartment. As expected, CD4Cre;miR-148a^{TG/+} mice displayed increased levels of miR-148a in splenic T cells compared to their control counterparts (**Figure 1B**).

### Example 2. 2. miR-148a expression under the control of the CD4Cre allele has an antitumoral effect in lung cancer and melanoma

Using the Lewis Lung Carcinoma (LLC) murine model, a drastic reduction in tumor growth was observed in CD4Cre;miR-148a^{TG/TG} (or CD4Cre;miR-148a^{fl/fl}) mice compared with their control counterparts (Figure 2A-B). In addition, significant reduction in tumor-infiltrating CD4 T cells was observed in mice with increased T cell expression of miR-148a (Figure 2C-D), demonstrating its immunomodulatory function in a tumor context.

The inventors also demonstrated an antitumor role for miR-148a in a preclinical mouse model of melanoma (B16-F10) (**Figure 3A**) with a significant reduction in tumor-infiltrating CD4 T cells (Figure 3B-C).

These results suggest a possible use of miR-148a as an agent to increase the efficiency of current immunotherapies, which could benefit a greater number of patients.

## Claims

1. miR-148a, miR-148a mimic, genetic construct encoding miR-148a and/or viral vector encoding miR-148a for use in the stimulation of T-cell anti-tumor response.

2. miR-148a, miR-148a mimic, genetic construct comprising the miR-148a gene and/or viral vector comprising the miR-148a gene for use, according to claim 1, wherein the viral vector is an adeno-associated viral vector, a retroviral vector or a lentiviral vector.

3. miR-148a, miR-148a mimic, genetic construct encoding miR-148a and/or viral vector encoding miR-148a for use, according to any of the previous claims, **characterized in that** the miR-148a, miR-148a mimic, genetic construct encoding miR-148a gene and/or viral vector encoding miR-148a is administered alone or prior, after or concomitantly to the administration a cancer treatment.

4. miR-148a, miR-148a mimic, genetic construct encoding miR-148a and/or viral vector encoding miR-148a for use, according to any of the previous claims, **characterized in that** the miR-148a, miR-148a mimic, genetic construct encoding miR-148a and/or viral vector encoding miR-148a is administered alone or prior, after or concomitantly to the administration a cancer treatment selected from the list comprising: checkpoint inhibitors, T-cell adoptive cell therapies, chemotherapy, radiotherapy, antibody-based cancer therapies, targeted-molecule inhibitors or small-molecule inhibitors.

5. miR-148a, miR-148a mimic, genetic construct encoding miR-148a and/or viral vector encoding miR-148a for use, according to any of the previous claims, in the treatment of lung cancer or melanoma.

6. miR-148a, miR-148a mimic, genetic construct encoding miR-148a and/or viral vector encoding miR-148a gene for use, according to any of the previous claims, **characterized in that** it is administered to the subject via intravenous administration, subcutaneous administration, intratumoral administration, or chemoembolization.

7. Composition comprising miR-148a, a miR-148a mimic, a genetic construct encoding miR-148a gene and/or a viral vector encoding miR-148a gene for use in the stimulation of T-cell anti-tumor response.

8. Composition comprising miR-148a, a miR-148a mimic, a genetic construct encoding miR-148a and/or a viral vector encoding miR-148a for use, according to claim 7, wherein the viral vector is an adeno-associated viral vector, a retroviral vector or a lentiviral vector.

9. Composition comprising miR-148a, a miR-148a mimic, a genetic construct encoding miR-148a and/or a viral vector encoding miR-148a for use, according to any of the claims 7 or 8, **characterized in that** the miR-148a, miR-148a mimic, genetic construct encoding miR-148a gene and/or viral vector encoding miR-148a is administered alone or prior, after or concomitantly to the administration a cancer treatment.

10. Composition comprising miR-148a, a miR-148a mimic, a genetic construct encoding miR-148a and/or a viral vector encoding miR-148a for use, according to any of the claims 7 to 9, **characterized in that** the miR-148a, miR-148a mimic, genetic construct encoding miR-148a and/or viral vector encoding miR-148a is administered alone or prior, after or concomitantly to the administration a cancer treatment selected from the list comprising: checkpoint inhibitors, T-cell adoptive cell therapies, chemotherapy, radiotherapy, antibody-based cancer therapies, targeted-molecule inhibitors or small-molecule inhibitors.

11. Composition comprising miR-148a, a miR-148a mimic, a genetic construct encoding miR-148a and/or a viral vector encoding miR-148a for use, according to any of the claims 7 to 10, in the treatment of lung cancer or melanoma.

12. Composition comprising miR-148a, a miR-148a mimic, a genetic construct encoding miR-148a and/or a viral vector encoding miR-148a for use, according to any of the claims 7 to 11, **characterized in that** it is administered to the subject via intravenous administration, subcutaneous administration, intratumoral administration, or chemoembolization.

13. Composition for use, according to any of the claims 7 to 12, **characterised in that** it is a pharmaceutical composition comprising pharmaceutically acceptable excipients or carriers.
